(19) Europäisches Patentamt
European Patent Office
Office européen des brevets

(11) Publication number: **0 097 978**
**B1**

(12) **EUROPEAN PATENT SPECIFICATION**

(45) Date of publication of patent specification: **18.12.85**

(51) Int. Cl.⁴: **C 07 C 51/12**, C 07 C 53/08, C 07 C 53/122, C 07 C 53/124

(21) Application number: **83200745.4**

(22) Date of filing: **25.05.83**

(54) **A process for the co-production of carboxylic acids.**

(30) Priority: **18.06.82 NL 8202493**

(43) Date of publication of application:
**11.01.84 Bulletin 84/02**

(45) Publication of the grant of the patent:
**18.12.85 Bulletin 85/51**

(84) Designated Contracting States:
**BE DE FR GB IT NL**

(56) References cited:
**GB-A-1 468 940**
**GB-A-1 523 346**
**GB-A-1 538 782**
**US-A-3 665 034**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Drent, Eit**
**Badhuisweg 3**
**NL-1031 CM Amsterdam (NL)**

(74) Representative: **Aalbers, Onno et al**
**P.O. Box 302**
**NL-2501 CH The Hague (NL)**

**EP 0 097 978 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to a process for the co-production of carboxylic acids and carboxylic acids having one carbon atom more in the molecule. The invention in particular relates to the co-production of acetic acid and propionic acid from methyl acetate, dimethyl ether, acetic anhydride or ethylidene diacetate.

US patent specification 4,111,982 gives a description of a process for the co-production of acetic acid and propionic acid by the reaction of methanol, ethylene, carbon monoxide and water in the presence of a rhodium compound and iodine or an iodine compound. One of the starting materials used in this process, ethylene, is a compound which is usually produced from petroleum, whereas interest tends towards a method in which all the starting materials are based on coal (synthesis gas).

From US patent specification 3,769,329 it is known that carboxylic acids can be obtained by carbonylation of alcohols. The carbonylation is carried out by reacting the alcohol with CO in the presence of a rhodium compound and bromine or iodine or a bromine compound or an iodine compound, optionally in the presence of hydrogen. If the reaction is carried out in the presence of water, the starting material used may be not alcohol, but an ester or ether derived therefrom. The carboxylic acid formed contains one carbon atom more than the alcohol or the alcohol-derived moiety present in the ester or ether. Thus the carbonylation for instance of methanol or methyl acetate according to US patent specification 3,769,329 leads to the formation of acetic acid. No mention is made in this specification of the formation of propionic acid.

It has now been found that if the above-mentioned rhodium-catalysed conversion of methylacetate or dimethyl ether is carried out under almost anhydric conditions in the presence of hydrogen and at least 2 moles of a carboxylic acid per mole of the starting compound, this will lead to the co-production of acetic acid and propionic acid. In addition, small quantities of normal and isobutyric acid may also be formed.

The invention relates to a process for the co-production of carboxylic acids of the formula $R^1COOH$ and/or $R^2COOH$ and carboxylic acids of the formula $R^1CH_2COOH$ and/or $R^2CH_2COOH$ by reacting one or more compounds of the formula $R^1XR^2$ in which X represents a

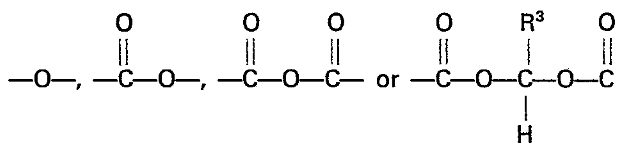

moiety and in which $R^1$, $R^2$ and $R^3$ represent similar or dissimilar alkyl, cycloalkyl, aryl, aralkyl or alkaryl groups, with carbon monoxide and hydrogen in the presence of a rhodium catalyst and an iodide and/or bromide source, characterized in that the reaction is carried out under virtually anhydric conditions and in the presence—per mole of the compound $R^1XR^2$—of at least 2 moles of a carboxylic acid of the formula $R^4COOH$, in which $R^4$ represents an alkyl, cycloalkyl, aryl, aralkyl or alkaryl group.

The carbonylation of methyl acetate or dimethyl ether under almost anhydric conditions has been described in the literature, but owing to the use of different reaction conditions, the products obtained therein are different from those obtained in the process according to the invention. For instance, the preparation of acetic anhydride by the reaction of methyl acetate or dimethyl ether with CO in the presence of methyl iodide and a compound of a Group VIII metal is known from UK patent specification 1,468,940. The reaction is carried out under anhydric conditions and, as can be seen from the examples, in the absence of carboxylic acids. In this patent specification no mention is made of the formation of acetic acid or propionic acid.

UK patent specification 1,523,346 relates to a similar process for the preparation of acetic anhydride by carbonylation of methyl acetate. It is stated that the presence of hydrogen can have a favourable effect on the course of the reaction, since it suppresses the formation of soot and carbon dioxide, but that it stimulates the formation of the by-product acetic acid. However, example 13 shows that the quantity of acetic acid formed in the presence of hydrogen is considerably smaller than the quantity used in the process according to the present invention; no mention is made of the formation of propionic acid.

From UK patent specification 1,538,782 it is known that ethylidene diacetate can be prepared by reaction of methyl acetate and/or dimethyl ether with carbon monoxide and hydrogen under anhydric conditions in the presence of a Group VIII noble metal catalyst, a bromide or an iodide and a promoter. This process leads to the formation of acetic anhydride, acetaldehyde and acetic acid as by-product, but the quantity of acetic acid formed is smaller than the minimum required in the process according to the present invention to bring about the formation of propionic acid. Further it is seen from Examples V and VIII of UK patent specification 1,538,782 that for obtaining reaction products the presence of a promoter is essential; in the process according to the invention this is not the case.

It is not known what mechanism governs the reaction or reactions involved in the process according to the invention. The "overall" reaction which takes place when methyl acetate is the starting material may be represented by the following reaction equation:

$$CH_3COOCH_3+2CO+2H_2 \rightarrow CH_3CH_2COOH+CH_3COOH.$$

When dimethyl ether, ethylidene diacetate or acetic anhydride is the starting material, the respective reaction equations will be:

$$CH_3OCH_3+3CO+2H_2 \rightarrow CH_3CH_2COOH+CH_3COOH,$$

$$CH_3CH(OCOCH_3)_2+2CO+3H_2 \rightarrow 2CH_3CH_2COOH+CH_3COOH,$$

$$CH_3COOCOCH_3+CO+2H_2 \rightarrow CH_3CH_2COOH+CH_3COOH.$$

Independent of each other, the hydrocarbon groups $R^1$, $R^2$, $R^3$ and $R^4$ represent alkyl, cycloalkyl, aryl, aralkyl or alkaryl groups. Preferably the hydrocarbon groups are unsubstituted and preferably they contain 1—20, in particular 1—6 carbon atoms.

Naturally, the composition of the reaction product obtained in the process according to the invention is dependent on the $R^1XR^2$ compound chosen. In order to keep this composition as simple as possible, the compound $R^1XR^2$ preferably used is one in which the groups $R^1$ and $R^2$ are identical. Examples of compounds of this type that are particularly preferred are methyl acetate, dimethyl ether, ethylidene acetate and acetic anhydride.

If compound $R^1XR^2$ is an ester, it should be taken into account that, as a side reaction, some ester interchange between the ester and the carboxylic acid $R^4COOH$ may occur. Therefore it is preferred to use a carboxylic acid $R^4COOH$ which corresponds with the acid-derived moiety of the ester. Thus, the reaction between, for instance, methyl acetate, CO and $H_2$ is preferably carried out in acetic acid.

If in the compound $R^1XR^2$ moiety X represents the group

—O—, 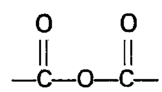 or 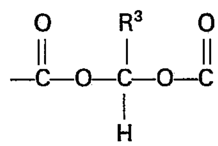

then preference is given to the use of a carboxylic acid $R^4COOH$ in which group $R^4$ is identical to $R^1$ or $R^2$, which groups are preferably identical as well, as mentioned hereinbefore.

The molar ratio of the carboxylic acid $R^4COOH$ to compound $R^1XR^2$ generally lies between 2:1 and 20:1, preferably between 3:1 and 15:1 and most preferably between 4:1 and 15:1. However, larger quantities of the carboxylic acid $R^4COOH$ can be used.

The rhodium catalyst may be of any suitable type. Suitable catalysts are, for instance, rhodium oxide and rhodium hydroxide, rhodium salts of mineral acids, such as hydrogen chloride, nitric acid and sulphuric acid and rhodium salts of organic acids, preferably of alkane carboxylic acids having 1—20 carbon atoms. The rhodium may also be complexed in a zero-valent form with ligands such as carbon monoxide, acetylacetone or organic compounds of nitrogen, phosphorus, arsenic or antimony.

The quantity of rhodium catalyst preferably lies between 0.01 and 10, in particular between 0.05 and 5% m per mole of compound $R^1XR^2$. Larger or smaller quantities can, however, be used.

The iodide or bromide source may be elementary iodine or bromine, a compound $R^5I$ or $R^5Br$, in which the group $R^5$ is preferably an alkyl group with 1—12 carbon atoms or a cycloalkyl, aryl, aralkyl or alkaryl group having not more than 12 carbon atoms, or an acyl iodide or acyl bromide $R^5$ COI or $R^5COBr$ in which $R^5$ has the above-mentioned meaning, hydrogen iodide or hydrogen bromide an alkali metal iodide or an alkaline earth metal iodide, for instance lithium iodide or sodium iodide or an alkali metal bromide or an alkaline earth metal bromide. If desired, two or more iodide and/or bromide sources may be used.

The quantity of the iodide or bromide source added to the reaction mixture is not critical. The number of gram atoms of iodine or bromine added in the elementary form or as iodide or bromide lies, for instance, between 0.1 and 200, preferably between 5 and 50 gram atoms per gram atom of rhodium.

The process according to the invention is preferably carried out in the presence of one or more promoters. Suitable promoters are the oxides of amines, or oxides, sulfides and selenides of phosphines, arsines and stibines. A group of promoters used by preference are those having the formula YZR′R″R‴, in which Y represents oxygen, sulphur or selenium, Z represents phosphorus, antimony or arsenic and the moieties R′, R″, R‴ independent of one another each represents a hydrogen atom or an alkyl, cycloalkyl, aryl or aralkyl group. These groups may be substituted, if required, with one or more substituents which under the reaction conditions are inert for instance, halogen atoms. The moieties R″ and R‴ together may represent an alkylene group which preferably has not more than 20 carbon atoms. Each alkyl group preferably contains not more than 20 carbon atoms, each cycloalkyl group preferably contains not more than 7 carbon atoms and each aryl group is preferably a phenyl group. Promoters in which R′, R″ and R‴ independent of one another represent an aryl group or a phenyl group are specially preferred. The moieties R′, R″ and R‴ preferably are identical, Z preferably represents a phosphorus atom. Oxides, sulphides and selenides of phosphines, arsines or stibines containing two or more phosphorus, arsenic or antimony atoms are also suitable as promoters.

Another group of suitable promoters is made up of compounds of the formula YZ(OR′) (OR″) (OR‴), in

which Y, Z, R', R'' and R''' have the meanings stated hereinbefore. This group includes for instance esters of phosphoric acid and thiophosphoric acid. Also suitable are compounds of the formula YZ $[(O)_aR']$ $[(O)_bR'']$ $[(O)_cR''']$, in which Y, Z, R', R'' and R''' have the meanings stated hereinbefore, each of a, b and c is 0 or 1, and a+b+c equals 1 or 2.

Examples of suitable promoters are trimethylphosphine oxide, tri - n - butylphosphine oxide, triphenylphosphine oxide, tri - p - tolylphosphine oxide, tetraphenyl dimethylene diphosphine dioxide (diphosdioxide), tetraphenyl trimethylene diphosphine dioxide, tri - n - butyl phosphate, triphenyl phosphate, dimethyl methylphosphonate, diphenyl methylphosphonate, methyl dimethylphosphonate, methyl diethylphosphonate and methyl diphenylphosphonate, trimethylarsine oxide, tri - isopropylstibine oxide, tricyclohexylarsine oxide, triphenylstibine oxide, diphenylethylarsine oxide, ethylenebis - (diphenylarsine) oxide, triphenylphosphine sulphide and tributylphosphine sulphide. Examples of suitable N-amine oxides are pyridine N-oxide, the picoline N-oxides and trimethylamine N-oxide.

Complexes which may be formed by the reaction of oxides, sulphides or selenides of phosphines, arsines or stibines with alkyl halides or hydrogen halides, such as

$$[(C_2H_5)_3AsO{-}H{-}OAs(C_2H_5)_3]^+I^- \text{ or } [(C_6H_5)_3PO{-}H{-}OP(C_6H_5)_3]^+I_3^-$$

are also suitable promoters.

The above-mentioned promoters are usually used in catalytic quantities. These quantities are not critical. Suitable quantities lie between, for example, 0.1 and 100, preferably between 0.5 and 10 mole per gram atom of rhodium.

Further, the process may suitably be carried out in the presence of small quantities—for instance of up to 100 equivalents per gram atom of rhodium—of a strong acid as the promoter. Suitable strong acids are those which, in an aqueous solution, at 20°C have a pKa lower than 3.5, for instance p-toluenesulphonic acid or trifluoromethanesulphonic acid or mineral acids, such as hydrochloric acid, sulphuric acid or perchloric acid.

The process is carried out under virtually anhydric conditions. However, such small quantities of water as may be present in commercially available starting materials, e.g. rhodium (III) chloride trihydrate, are permissible. The reaction mixture preferably contains not more than 2, in particular not more than 0.2%w water.

The process according to the invention may be carried out either in the liquid phase or in the gaseous phase. The hydrogen and the carbon monoxide may be added simultaneously if desired. Although usually carbon monoxide and hydrogen are added in more or less equimolar quantities, the molar ratios between the two compounds may vary within wide limits, for instance in the range of from 10:1 to 1:10. The carbon monoxide:hydrogen molar ratio preferably lies between 1:0.5 and 1:3. Inert gases, such as nitrogen, noble gases, carbon dioxide or methane may optionally be present in the reaction mixture. The process may be carried out batchwise, continuously or semi-continuously.

The process according to the invention is preferably carried out at a temperature between 50 and 300°C, in particular between 50 and 200°C and most preferably between 125 and 175°C. Either low pressure of, for instance, 5 bar or high pressures of, for instance, 1000 bar may be used. Usually high pressures are not economical on account of the investment and energy costs involved. The pressure preferably lies between 20 and 100 bar.

The process according to the invention may be carried out in the presence of an additional solvent, if desired. However, compound $R^1XR^2$, such as methyl acetate, ethyl acetate, methyl propionate, ethyl propionate, dimethyl ether, diethyl ether or methyl t-butyl ether and/or the carboxylic acid $R^4COOH$, for instance acetic acid or propionic acid, may in itself serve as a solvent as well. In that case it should naturally be ensured that the carboxylic acid $R^4COOH$ is present in such a proportion to compound $R^1XR^2$ as is required according to the invention. Suitable additional solvents include cyclic ethers, such as tetrahydrofuran, 1,3-dioxane, 1,4-dioxane and the dioxolanes, sulphones and sulphoxides, such as dimethyl sulphone, sulpholane, 2-methyl sulpholane, 3-methyl sulpholane, dimethyl sulphoxide and diethyl sulphoxide. Butyrolacetone is a very suitable solvent. The additional solvent is generally used in quantities sufficient to keep the reaction mixture homogeneous—for instance 5—20 %v, calculated on the total reaction mixture.

The reaction mixture obtained in the process according to the invention may be processed by known techniques, such as fractional distillation. If desired, the products may be subjected to further purification treatments.

With respect both to the starting materials and to conversion of the acids formed, the process may be integrated into existing processes. For instance, the process is excellently suitable for the co-production of acetic acid and propionic acid from methyl acetate which in its turn can be prepared by carbonylation of methanol. Whilst the propionic acid obtained may be of interest in itself, the acetic acid formed as a by-product can be esterified with methanol to form methyl acetate, which compound can be recirculated to the reactor.

Example I

The process was carried out in a 300 ml magnetically stirred Hastalloy C autoclave (Hastelloy is a trade

mark) containing 35 ml (0.6 mole) acetic acid and 1 mmole rhodium (III) chloride trihydrate and methylacetate, methyl iodide or lithium iodide and optionally triphenylphosphine, triphenylphosphine oxide, dimethyl methylphosphonate or p-toluenesulphonic acid in the quantities given in Table A. The autoclave was flushed with carbon monoxide and then filled with a mixture of carbon monoxide and hydrogen (molar ratio 1:1) under pressure. The temperatures and pressures used are given in Table A. The autoclave was kept at the temperature mentioned for 15 hours. Subsequently the reaction mixture was cooled and analysed by means of gas-liquid chromatography. The methyl acetate conversion was unvariably 100%. The yields of propionic acid and of normal and isobutyric acid are given in Table A in %m, calculated on methyl acetate converted. All the experiments in addition yielded acetic acid as a co-product in at least equimolar quantities calculated on propionic acid produced.

TABLE A

| Exp. No. | Iodide-source mmole | Promoter mmole | Methyl-acetate mole | Temp. °C | Pressure bar | Yield of propionic acid % m | Yield of i+n butyric acid % m |
|---|---|---|---|---|---|---|---|
| 1 | MeI (30) | — | 0.2 | 145 | 40 | 33 | — |
| 2 | MeI (30) | — | 0.1 | 145 | 40 | 40 | — |
| 3 | MeI (30) | p-TS(3) | 0.2 | 150 | 40 | 47 | 5 |
| 4 | MeI (30) | — | 0.05 | 145 | 40 | 44 | — |
| 5 | MeI (30) | p-TS(3) | 0.1 | 150 | 60 | 60 | 5—10 |
| 6 | MeI (30) | TPPO(4) | 0.1 | 160 | 60 | 72 | 5—10 |
| 7 | MeI (15) | TPPO(4) | 0.1 | 160 | 60 | 78 | 10—15 |
| 8 | LiI | p-TS(3) | 0.1 | 150 | 60 | 10 | — |
| 9 | MeI (7.5) | TPPO(4) | 0.1 | 160 | 60 | 72 | 10—15 |
| 10 | MeI (15) | DMMP(4) | 0.1 | 160 | 60 | 60 | 5—10 |
| Comparative experiments | | | | | | | |
| 11 | MeI (30) | — | 0.4 | 160 | 40 | trace | — |
| 12* | MeI (30) | p-TS(3) | 0.1 | 140 | 60 | trace | — |
| 13** | MeI (30) | p-TS(3) | 0.1 | 155 | 40 | trace | — |
| 14*** | MeI (15) | TPPO(4) | 0.1 | 160 | 60 | trace | — |

MeI=methyl iodide
p-TS=p-toluenesulphonic acid
TPPO=triphenylphosphine oxide
DMMP=dimethyl methylphosphonate

* In this experiment not 35 ml acetic acid but 35 ml sulpholane was used.

** In this experiment not 1 mmol rhodium (III) chloride trihydrate but 1 mmole ruthenium (III) chloride trihydrate was used.

*** In this experiment not 1 mmole rhodium chloride trihydrate but 0.7 mmole palladium diacetate was used.

Comparison of Experiments 1 and 2 shows that raising the acetic acid:methyl acetate molar ratio from 3:1 to 6:1 increases the yield of propionic acid. Experiment 11 shows that when said molar ratio is lower than 2:1, no propionic acid is formed. Experiment 12 shows that not even the presence of the active promoter p-toluenesulphonic acid allows propionic acid to be formed in the absence of acetic acid. Experiments 13 and 14 show that under the reaction conditions used compounds of Ru and Pd do not catalyse the desired conversion.

Example II

The process was carried out in a 300 ml magnetically stirred Hastelloy C autoclave (Hastelloy is a trade mark) containing 35 ml of an alkane carboxylic acid, 1 mmole rhodium (III) chloride trihydrate, 15 mmole methyl iodide and 4 mmole triphenylphosphine oxide, and 7.5 ml methyl acetate, methyl propionate, ethyl propionate, acetic anhydride or ethylidene diacetate. The autoclave was flushed with carbon monoxide and subsequently filled with a mixture of carbon monoxide and hydrogen (molar ratio 1:1) at a pressure of 60 bar. The autoclave was kept at a temperature of 160°C for 15 hours. Then the reaction mixture was cooled and analysed by gas-liquid chromatography. Conversion was 100% in the experiments using methyl acetate, methyl propionate, acetic anhydride and ethylidene diacetate and 80% in the experiment using ethyl propionate. The alkane carboxylic acids and the concentrations in which the various carboxylic acids were present in the reaction mixture are given in Table B.

TABLE B

| | Alkane carboxylic acid | Carboxylic acid concentrations in reaction mixture, %w | |
|---|---|---|---|
| methyl acetate | propionic acid | acetic acid | 19 |
| | | propionic acid | 67 |
| | | n-butyric acid | 6.3 |
| | | isobutyric acid | 4.9 |
| methyl propionate | acetic acid | acetic acid | 74 |
| | | propionic acid | 22 |
| | | n-butyric acid | 1.3 |
| | | isobutyric acid | 1.2 |
| methyl propionate | propionic acid | acetic acid | 9 |
| | | propionic acid | 78 |
| | | n-butyric acid | 6 |
| | | isobutyric acid | 4.5 |
| ethyl propionate | propionic acid | propionic acid | 87 |
| | | n-butyric acid | 3.5 |
| | | isobutyric acid | 2.6 |
| acetic anhydride | acetic acid | acetic acid | 87 |
| | | propionic acid | 9.5 |
| | | n-butyric acid | 0.5 |
| | | isobutyric acid | 0.5 |
| ethylidene diacetate | acetic acid | acetic acid | 85 |
| | | propionic acid | 13.1 |
| | | n-butyric acid | 1.0 |
| | | isobutyric acid | 1.0 |

**Claims**

1. A process for the co-production of carboxylic acids of formula $R^1COOH$ and/or $R^2COOH$ and carboxylic acids of the formula $R^1CH_2COOH$ and/or $R^2CH_2COOH$ by reacting one or more compounds of the formula $R^1XR^2$ in which X represents a

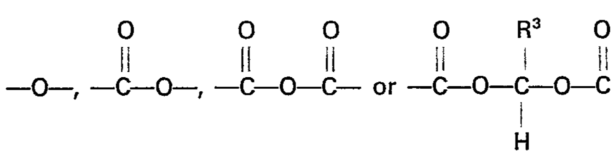

moiety and in which $R^1$, $R^2$ and $R^3$ represent similar or dissimilar alkyl, cycloalkyl, aryl, aralkyl or alkaryl groups, with carbon monoxide and hydrogen in the presence of a rhodium catalyst and an iodide and/or bromide source, characterized in that the reaction is carried out under virtually anhydric conditions and in the presence—per mole of the compound $R^1XR^2$—of at least 2 moles of a carboxylic acid of the formula $R^4COOH$, in which $R^4$ represents an alkyl, cycloalkyl, aryl, aralkyl or alkaryl group.

2. A process as claimed in claim 1, characterized in that the groups $R^1$, $R^2$, $R^3$ and $R^4$ contain 1—20 carbon atoms.

3. A process as claimed in claim 1 or 2, characterized in that the groups $R^1$ and $R^2$ are identical.

4. A process as claimed in claim 3, characterized in that the compound $R^1XR^2$ is methyl acetate, dimethyl ether, ethylidene diacetate or acetic anhydride.

5. A process as claimed in claim 1—4, characterized in that when compound $R^1XR^2$ is an ester, the carboxylic acid $R^4COOH$ corresponds with the acid-derived moiety of the ester and when in compound $R^1XR^2$ the moiety X represents

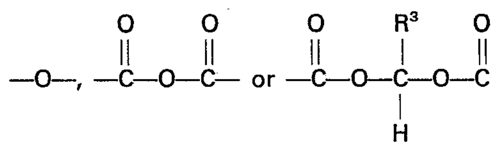

the group $R^4$ of carboxylic acid $R^4COOH$ is identical with $R^1$ or $R^2$.

6. A process as claimed in claims 1—5, characterized in that the molar ratio of carboxylic acid $R^4COOH$ to compound $R^1XR^2$ lies between 2:1 and 20:1.

7. A process as claimed in claim 5, characterized in that the molar ratio of carboxylic acid $R^4COOH$ to compound $R^1XR^2$ lies between 3:1 and 15:1, in particular between 4:1 and 15:1.

8. A process as claimed in claims 1—7, characterized in that the quantity of rhodium catalyst lies between 0.01 and 10 moles per mole of compound $R^1XR^2$.

9. A process as claimed in claim 1—8, characterized in that per gram atom of rhodium 0.1—200 gram atom of iodine and/or bromium in elementary form or as iodide and/or bromide is present in the reaction mixture.

10. A process as claimed in claims 1—9, characterized in that the process is carried out in the presence of a compound having the formula YZR'R''R''', YZ (OR') (OR'') (OR''') or $YZ[(O)_aR'] [(O)_bR''] [(O)_cR''']$, in which Y is oxygen, sulphur or selenium, Z represents phosphorus, antimony or arsenic and the moieties R', R'' and R''' independent of one another, each represent a hydrogen atom or an alkyl, cycloalkyl, aryl or aralkyl group which may be substituted with an inert substituent, a, b and c each are 0 or 1, and a+b+c equals 1 or 2 as promoter.

11. A process as claimed in claim 10, characterized in that the quantity of promoter lies between 0.1 and 100 mole per gram atom of rhodium.

12. A process as claimed in claims 1—9, characterized in that the process is carried out in the presence of up to 100 equivalents per gram atom of rhodium, of an acid which, in an aqueous solution, at 20°C has a pKa of less than 3.5.

13. A process as claimed in claims 1—12, characterized in that the process is carried out at a temperature in the range between 50 and 300°C and a pressure in the range between 20 and 100 bar.

**Patentansprüche**

1. Ein Verfahren zur gleichzeitigen Herstellung von Carbonsäuren der Formel $R^1COOH$ und/oder $R^2COOH$ und von Carbonsäuren der Formel $R^1CH_2COOH$ und/oder $R^2CH_2COOH$ durch Umsetzen einer oder mehrerer Verbindungen der Formel $R^1XR^2$, in welcher X eine

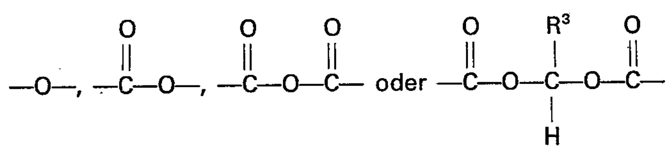

gruppe derstellt und in welcher $R^1$, $R^2$ und $R^3$ Alkyl-, Cycloalkyl, Aryl-, Aralkyl- oder Alkarylgruppen, welche gleich oder verschieden sein können, darstellen, mit Kohlenmonoxid und Wasserstoff in Gegenwart eines Rhodium-Katalysators und einer Jod und/oder Bromquelle, dadurch gekennzeichnet, daß die Reaktion unter praktisch wasserfreien Bedingungen und in Gegenwart von mindestens 2 Molje Mol der Verbindung $R^1XR^2$—einer Carbonsäure der Formel $R^4COOH$, in welcher $R^4$ eine Alkyl-, Cycloalkyl-, Aryl-, Aralkyl- oder Alkarylgruppe bedeutet, durchgeführt wird.

2. Ein Verfahren wie in Anspruch 1 beansprucht, dadurch gekennzeichnet, daß die Gruppen $R^1$, $R^2$, $R^3$ und $R^4$ 1 bis 20 Kohlenstoffatome enthalten.

3. Ein Verfahren wie in Anspruch 1 oder 2 beansprucht, dadurchgekennzeichnet, daß die Gruppen $R^1$ und $R^2$ identisch sind.

4. Ein Verfahren wie in Anspruch 3 beansprucht, dadurch gekennzeichnet, daß die Verbindung $R^1$ $XR^2$ Methylacetat, Dimethyläther, Ethyliden-diacetat oder Essigsäureanhydrid ist.

5. Ein Verfahren wie in den Ansprüchen 1 bis 4 beansprucht, dadurch gekennzeichnet, daß, wenn die

Verbindung $R^1XR^2$ ein Ester ist, die Carbonsäure $R^4COOH$ dem Säurerest des Esters entspricht, und wenn die Gruppe X in der Verbindung $R^1XR^2$

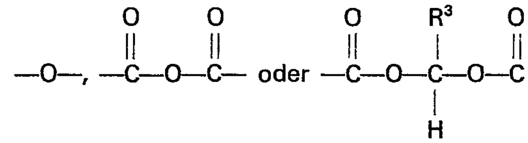

ist, die Gruppe $R^4$ der Carbonsäure $R^4COOH$ mit $R^1$ oder $R^2$ identisch ist.

6. Ein Verfahren wie in den Ansprüchen 1 bis 5 beansprucht, dadurch gekennzeichnet, daß das molare Verhältnis von Carbonsäure $R^4COOH$ zu Verbindung $R^1XR^2$ zwischen 2:1 und 20:1 liegt.

7. Ein Verfahren wie in Anspruch 5 beansprucht, dadurch gekennzeichnet, daß das molare Verhältnis der Carbonsäure $R^4COOH$ zur Verbindung $R^1XR^2$ zwischen 3:1 und 15:1, und bevorzugt zwischen 4:1 und 15:1 liegt.

8. Ein Verfahren wie in den Ansprüchen 1 bis 7 beansprucht, dadurch gekennzeichnet, daß die Menge des Rhodium-Katalysators zwischen 0,01 und 10 Mol je Mol der Verbindung $R^1XR^2$ beträgt.

9. Ein Verfahren wie in den Ansprüchen 1 bis 8 beansprucht, dadurch gekennzeichnet, daß je Grammatom Rhodium 0,1 bis 200 Grammatom Jod und/oder Brom als Elemente or als Jodid und/oder Bromid in der Reaktionsmischung vorliegen.

10. Ein Verfahren wie in den Ansprüchen 1 bis 9 beansprucht, dadurch gekennzeichnet, daß das Verfahren in Gegenwart einer Verbindung mit den Formeln YZR'R''R''' YZ (OR') (OR'') (OR''') oder YZ $[(O)_aR']$ $[(O)_bR'']$ $[(O)_cR''']$, in welchen Y Sauerstoff, Schwefel oder Selen ist, Z Phosphor, Antimon oder Arsen bedeutet und die Gruppen R', R'' und R''' unabhängig voneinander je ein Wasserstoffatom oder eine Alkyl-, Cycloalkyl-, Aryl- oder Aralkylgruppe, welche durch einen inerten Substituenten substituiert sein kann, darstellen und in welcher a, b und c jeweils 0 oder 1 bedeuten, und a+b+c den Wert 1 oder 2 ergeben, als Promotor durchgeführt wird.

11. Ein Verfahren wie in Anspruch 10 beansprucht, dadurch gekennzeichnet, daß die Menge des Promotors zwischen 0,1 und 100 Mol je Grammatom Rhodium liegt.

12. Ein Verfahren wie in den Ansprüchen 1 bis 9 beansprucht, dadurch gekennzeichnet, daß das Verfahren in Gegenwart von bis zu 100 Grammäquivalenten, bezogen auf Rhodium, einer Säure, welche in einer wäßrigen Lösung bei 20°C einen pKa-Wert von weniger als 3,5 aufweist, durchgeführt wird.

13. Ein Verfahren wie in den Ansprüchen 1 bis 12 beansprucht, dadurch gekennzeichnet, daß das Verfahren bei einer Temperatur im Bereich zwischen 50 und 300°C und einem Druck im Bereich zwischen 20 und 100 bar durchgeführt wird.

**Revendications**

1. Un procédé pour la coproduction d'acides carboxyliques de formule $R^1COOH$ et/ou $R^2COOH$ et d'acides carboxyliques de formule $R^2CH_2COOH$ et/ou $R^2CH_2COOH$ en faisant réagir un ou plusieurs composés de la formule $R^1XR^2$ où X représente un portion

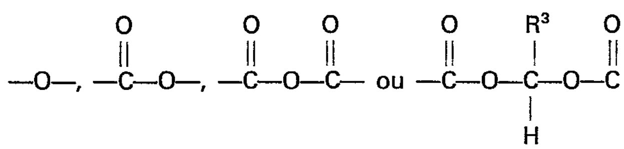

et $R^1$, $R^2$ et $R^3$ représentent des groupes alcoyle, cycloalcoyle, aryle, aralcoyle ou alcaryle identiques ou différents, avec l'oxyde de carbone et l'hydrogène en présence d'un catalyseur au rhodium et d'une source d'iodure et/ou de bromure, caractérisé en ce que la réaction est conduite dans des conditions pratiquement anhydres et en présence, par mole du composé $R^1XR^2$, d'au moins 2 moles d'un acide carboxylique de la formule $R^4COOH$, où $R^4$ représente un groupe alcoyle, cycloalcoyle, aryle, aralcoyle ou alcaryle.

2. Un procédé selon la revendication 1, caractérisé en ce que les groupes $R^1$, $R^2$, $R^3$ et $R^4$ contiennent 1—20 atomes de carbone.

3. Un procédé selon la revendication 1 ou 2, caractérisé en ce que les groupes $R^1$ et $R^2$ sont identiques.

4. Un procédé selon la revendication 3, caractérisé en ce que le composé $R^1XR^2$ est l'acétate de méthyle, l'oxyde de méthyle, le diacétate d'éthylidène ou l'anhydride acétique.

5. Un procédé selon les revendications 1—4, caractérisé en ce que quand le composé $R^1XR^2$ est un ester, l'acide carboxylique $R^4COOH$ correspond à la portion de l'ester dérivée d'un acide, et quand dans le composé $R^1XR^2$ la portion X représente

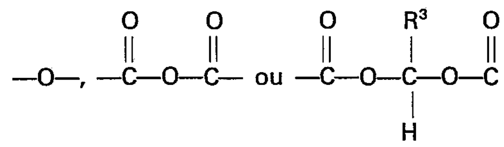

le groupe $R^4$ de l'acide carboxylique $R^4COOH$ est identique à $R^1$ ou $R^2$.

6. Un procédé selon les revendications 1—5, caractérisé en ce que le rapport molaire de l'acide carboxylique $R^4COOH$ au composé $R^1XR^2$ est compris entre 2:1 et 20:1.

7. Un procédé selon la revendication 5, caractérisé en ce que le rapport molaire de l'acide carboxylique $R^4COOH$ au composé $R^1XR^2$ est comprise entre 3:1 et 15:1, en particulier entre 4:1 et 15:1.

8. Un procédé selon les revendications 1—7, caractérisé en ce que la quantité de catalyseur au rhodium est comprise entre 0,01 et 10 moles par mole de composé $R^1XR^2$.

9. Un procédé selon les revendications 1—8, caractérisé en ce que par atome-gramme de rhodium, il y a dans le mélange réactionnel 0,1—200 atomes-grammes d'iode et/ou de brome dans la forme élémentaire ou sous la forme d'iodure et/ou de bromure.

10. Un procédé selon les revendications 1—9, caractérisé en ce que le procédé est mis en oeuvre en présence d'un composé ayant la formule YZR'R''R''', YZ (OR') (OR'') (OR''') ou $YZ[(O)_aR'][(O)_bR''][(O)_cR''']$, où Y est de l'oxygène, du soufre ou du sélénium, Z représente du phosphore, de l'antimoine ou de l'arsenic et les portions R', R'' et R''', indépendamment les unes des autres, représentent chacune un atome d'hydrogène ou un groupe alcoyle, cycloalcoyle, aryle ou aralcoyle qui peut être substitué par un substituant inerte, a, b et c sont chacun 0 ou 1 et a+b+c=1 ou 2 comme promoteur.

11. Un procédé selon la revendication 10, caractérisé en ce que la quantité de promoteur est comprise entre 0,1 et 100 moles par atome-gramme de rhodium.

12. Un procédé selon les revendications 1—9, caractérisé en ce que le procédé est mis en oeuvre en présence de jusqu'à 100 équivalents par atome-gramme de rhodium d'un acide qui, dans une solution aqueuse à 20°C, a un $pK_a$ de moins de 3,5.

13. Un procédé selon les revendications 1—12, caractérisé en ce que le procédé est mis en oeuvre à une température comprise entre 50 et 300°C et une pression comprise entre 20 et 100 bars.